(19) Europäisches Patentamt
European Patent Office
Office européen des brevets

(11) Veröffentlichungsnummer : **0 104 549**
**B1**

(12) # EUROPÄISCHE PATENTSCHRIFT

(45) Veröffentlichungstag der Patentschrift : **29.07.87**

(51) Int. Cl.⁴ : **C 07 D249/08**, C 07 D233/60, A 01 N 43/64, A 01 N 43/50

(21) Anmeldenummer : **83109103.8**

(22) Anmeldetag : **15.09.83**

(54) **Azolylbutanole.**

(30) *Priorität* : **29.09.82 DE 3235935**

(43) Veröffentlichungstag der Anmeldung : **04.04.84 Patentblatt 84/14**

(45) Bekanntmachung des Hinweises auf die Patenterteilung : **29.07.87 Patentblatt 87/31**

(84) Benannte Vertragsstaaten : **BE CH DE FR GB IT LI NL**

(56) Entgegenhaltungen :
**EP-A- 0 044 605**
**DE-A- 2 734 365**
**DE-A- 2 920 374**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Regel, Erik, Dipl.-Ing.**
**Untere Bergerheide 26**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Brandes, Wilhelm, Dr.**
**Eichendorffstrasse 3**
**D-5653 Leichlingen (DE)**
Erfinder : **Reinecke, Paul, Dr.**
**Lessingstrasse 11**
**D-5090 Leverkusen (DE)**

EP 0 104 549 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Azolylbutanole, mehrere Verfahren zu ihrer Herstellung und ihre Verwendung als Pflanzenschutzmittel.

Es ist bereits bekannt, daß bestimmte Azol-substituierte Carbinole, wie z. B. 2-(4-Chlorbiphenyl-4'-yl)-3-(4-chlorphenyl)-1-(1,2,4-triazol-1-yl)-propan-2-ol oder 2-Biphenyl-4-yl-3-(4-chlorphenyl)-1-(1,2,4-triazol-1-yl)-propan-2-ol fungizide Eigenschaften besitzen (vergleiche DE-A- 29 20 374).

Die Wirksamkeit dieser Verbindungen ist jedoch insbesondere bei niedrigen Aufwandmengen und -konzentrationen nicht immer ganz zufriedenstellend.

Weiterhin sind aus der DE-A-27 34 365 und der EP-A-0 044 605 zahlreiche Azolyl-Derivate mit fungiziden Eigenschaften bekannt. Es werden jedoch keine Verbindungen dieses Typs beschireben, in denen zwei gegebenenfalls substituierte Phenyl-Reste vorhanden sind, die jeweils über eine $CH_2$-Gruppe mit dem zentralen Kohlenstoffatom verbunden sind.

Es wurden nun neue Azolylbutanole der allgemeinen Formel (I),

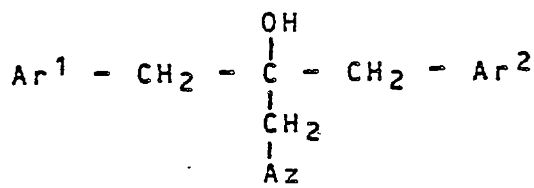
(I)

in welcher

Az für 1,2,4-Triazol-1-yl oder Imidazol-1-yl steht und

$Ar^1$ und $Ar^2$ unabhängig voneinander für Phenyl stehen, wobei die Phenylreste gegebenenfalls ein- bis dreifach substituiert sein könen durch Halogen, Alkyl oder Alkoxy mit jeweils 1 bis 4 Kohlenstoffatomen, Halogenalkyl mit 1 bis 2 Kohlenstoffatomen und 1 bis 5 Halogenatomen, sowie durch gegebenenfalls ein- bis dreifach gleich oder verschieden durch Alkyl mit 1 bis 4 Kohlenstoffatomen oder Halogen substituiertes Phenyl,

sowie deren pflanzenverträgliche Säureadditionssalze und Metallsalzkomplexe gefunden.

Weiterhin wurde gefunden, daß man die neuen Azolylbutanole der allgemeinen Formel (I) sowie deren pflanzenverträgliche Säureadditions-Salze und Metallsalz-Komplexe erhält, wenn man

a) Azolylpropanone der allgemeinen Formel (II),

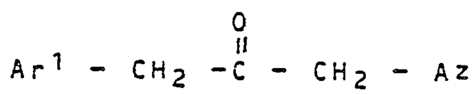
(II)

in welcher $Ar^1$ und Az die oben angegebene Bedeutung haben, mit magnesium-organischen Verbindungen der allgemeinen Formel (III b)

$$Ar^2—CH_2—Mg—Hal \qquad (III\ b)$$

in welcher

$Ar^2$ die oben angegebene Bedeutung hat und

Hal für Halogen steht,

in Gegenwart eines Verdünnungsmittels umsetzt, oder

b) substituierte Oxirane der allgemeinen Formel (IV),

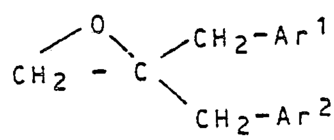
(IV)

in welcher $Ar^1$ und $Ar^2$ die oben angegebene Bedeutung haben, mit Alkalisalzen von Azolen der allgemeinen Formel (V),

$$Az^{\ominus}M^{\oplus} \qquad (V)$$

in welcher

Az die oben angegebene Bedeutung hat und
M für ein Alkalimetall steht,
in Gegenwart eines Verdünnungsmittels umsetzt, oder

c) Azolylessigsäureester der allgemeinen Formel (VI),

$$Az—CH_2—COOR$$

in welcher
Az die oben angegebene Bedeutung hat und
R für Alkyl steht,
mit magnesium-organischen Verbindungen der Formel (III a),

$$Ar^1—CH_2—Mg—Hal \qquad (IIIa)$$

in welcher
$Ar^1$ die oben angegebene Bedeutung hat und
Hal für Halogen steht,
in Gegenwart eines Verdünnungsmittels umsetzt, und gegebenenfalls an die so erhaltenen Verbindungen der Formel (I) anschließend eine Säure oder ein Metallsalz addiert.

Die neuen Azolylbutanole der Formel (I) weisen starke fungizide Eigenschaften auf. Dabei zeigen die erfindungsgemäßen Verbindungen überraschenderweise eine bessere fungizide Wirksamkeit als die nach dem Stand der Technik bekannten Verbindungen 2-(4-Chlorbiphenyl-4'-yl)-3-(4-chlorphenyl)-1-(1,2,4-triazol-1-yl)-propan-2-ol oder 2-Biphenyl-4-yl-3-(4-chlorphenyl)-1-(1,2,4-triazol-1-yl)-propan-2-ol, welche chemisch und wirkungsmäßig naheliegende Verbindungen sind.

Bevorzugt sind diejenigen Verbindungen der Formel (I), bei denen
Az für 1,2,4-Triazol-1-yl oder Imidazol-1-yl steht und
$Ar^1$ und $Ar^2$ unabhängig voneinander für gegebenenfalls ein- bis dreifach gleich oder verschieden substituiertes Phenyl stehen, wobei als Substituenten besonders bevorzugt sind : Fluor, Chlor, Brom, Methyl, Ethyl, Isopropyl, Methoxy, Ethoxy, Isopropyloxy, Trifluormethyl sowie gegebenenfalls durch Chlor oder Methyl ein- bis dreifach gleich oder verschieden substituiertes Phenyl.

Verwendet man beispielsweise 1-Phenyl-3-(1,2,4-triazol-1-yl)-propan-2-on und Benzylmagnesiumchlorid als Ausgangsstoffe, so kann der Reaktionsablauf des erfindungsgemäßen Verfahrens

a) durch das folgende Formelschema wiedergegeben werden :

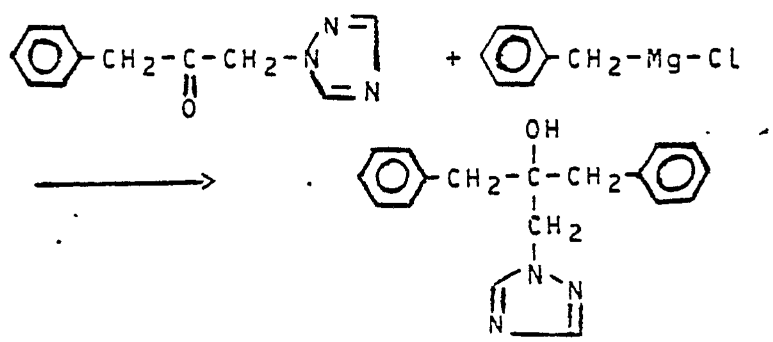

Verwendet man beispielsweise 1-(4-Chlorbenzyl)-1-(2,4-di-chlorbenzyl)-oxiran und das Natriumsalz des 1,2,4-Traizols als Ausgangsstoffe, so kann der Reaktionsablauf des erfindungsgemäßen Verfahrens b) durch das folgende Formelschema wiedergegeben werden :

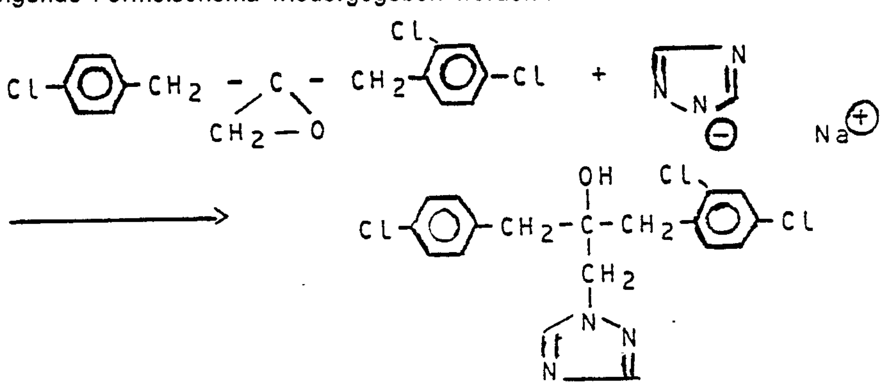

Verwendet man beispielsweise 4-Fluorbenzylmagnesiumchlorid und 1,2,4-Triazol-1-yl-essigsäure-methylester als Ausgangsstoffe so kann der Reaktionsablauf des erfindungsgemäßen Verfahren c) durch das folgende Formelschema wiedergegeben werden :

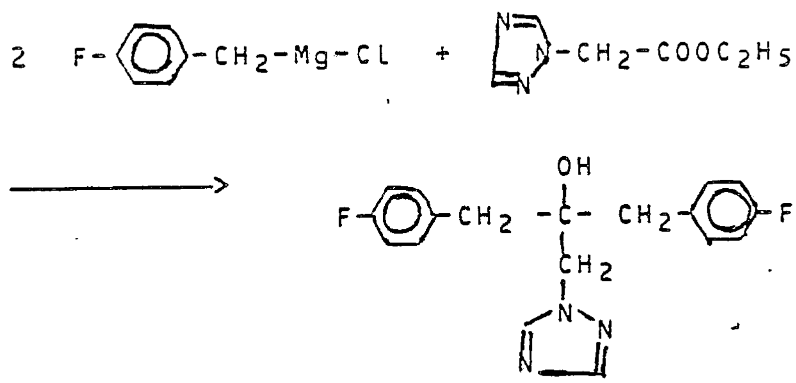

Die bei der Durchführung des erfindungsgemäßen Verfahrens a) als Ausgangsstoffe benötigten Azolylpropanone sind durch die Formel (II) allgemein definiert. In dieser Formel (II) besitzen $Ar^1$ und Az dieselbe vorzugsweise Bedeutung, die bei der Beschreibung der erfindungsgemäßen Stoffe der Formel (I) vorzugsweise für diese Reste genannt wurde.

Die Azolylpropane der Formel (II) sind in der DE-A-31 35 186 beschrieben. Man erhält sie z. B., indem man Arylacetonitrile der allgemeinen Formel (VII).

$$Ar^1—CH_2—CN \qquad (VII)$$

in welcher $Ar^1$ die oben angegebene Bedeutung hat, mit Azolylessigsäureestern der allgemeinen Formel (VI)

$$Az—CH_2—COOR \qquad (VI),$$

in welcher

Az die oben angegebene Bedeutung hat und

R für Alkyl mit 1 bis 4 Kohlenstoffatomen steht,

in Gegenwart eines Base, wie z. B. Natriummethylat, und in Gegenwart eines inerten organischen Lösungsmittels, wie z. B. Methanol, vorzugsweise bei der Siedetemperatur des verwendeten Lösungsmittels, umsetzt und anschließend im System Schwefelsäure/Wasser hydrolysiert, oder indem man Azolylessigsäureester der oben angegebenen Formel (VI) mit magnesium-organischen Verbindungen der allgemeinen Formel (IIIa)

$$Ar^1—CH_2—Mg—Hal \qquad (IIIa)$$

in welcher

$Ar^1$ die oben angegebene Bedeutung hat und

Hal für Chlor oder Brom steht,

in Gegenwart eines inerten organischen Lösungsmittels, wie z. B. Ether oder Tetrahydrofuran, bei Temperaturen zwischen 30 °C und 80 °C umsetzt.

Die weiterhin als Ausgangsstoffe zu verwendenden substituierten Oxirane sind durch die Formel (IV) allgemein definiert. In dieser Formel besitzen $Ar^1$ und $Ar^2$ dieselbe vorzugsweise Bedeutung, die bei der Beschreibung der erfindungsgemäßen Stofe der Formel (I) vorzugsweise für diese Reste genannt wurden.

Die substituierten Oxirane der Formel (IV) sind noch nicht bekannt. Man erhält sie z. B., indem man Arylacetonitrile der Formel (VII),

$$Ar^1—CH_2—CN \qquad (VII)$$

in welcher $Ar^1$ die oben angegebene Bedeutung hat, mit Arylessigsäureestern der Formel (VIII),

$$Ar^2—CH_2—COOR \qquad (VIII)$$

in welcher

$Ar^2$ die oben angegebene Bedeutung hat und

R für Alkyl mit 1 bis 4 Kohlenstoffatomen steht,

in Gegenwart einer Base, wie z. B. Natriummethylat und in Gegenwart eines inerten organischen

Lösungsmittels, wie z. B. Methanol, vorzugsweise bei der Siedetemperatur des verwendeten Lösungsmittels umsetzt, anschließend mit verdünnter Mineralsäure, wie z. B. Salzsäure hydrolysiert und die so erhaltenen Diarylpropanone der Formel (IX),

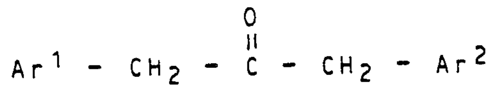 (IX)

in welcher $Ar^1$ und $Ar^2$ die oben angegebene Bedeutung haben entweder mit Dimethyloxosulfoniummethylid in an sich bekannter Weise in Gegenwart eines Verdünnungsmittels, wie beispielsweise Dimethylsulfoxid, bei Temperaturen zwischen 20 und 80 °C umsetzt; oder mit Trimethylsulfoniummethylsulfat in an sich bekannter Weise in Gegenwart eines Zweiphasensystems und gegebenenfalls in Gegenwart eines Phasentransferkatalysators bei Temperaturen zwischen 0 und 100 °C umsetzt.

Eine alternative Herstellungsmethode für die substituierten Oxirane der Formel (IV) besteht darin, daß man zunächst das bekannte 1,1-Bis-(chlormethyl)-oxiran (vergl. Beilstein Bd. 1 E III, S. 1587-1588) der Formel (XIII)

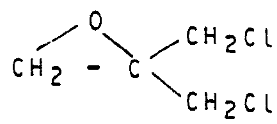 (XIII)

mit magnesium-organischen Verbindungen der Formel (III a) umsetzt zu den Chlorhydrinen der Formel (X),

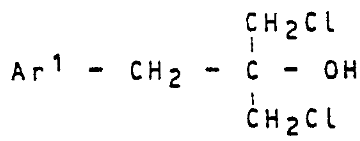 (X)

in welcher $Ar^1$ die oben angegebene Bedeutung hat, die Chlorhydrine der Formel (X) dann mit einer Base, wie z. B. Natriumhydroxid in bekannter Weise (vgl. J. org. Chemistry 27, 2242 [1961]) in die Oxirane der Formel (XI),

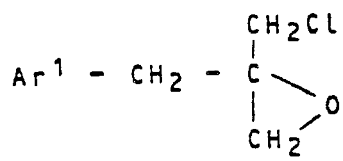 (XI)

in welcher $Ar^1$ die oben angegebene Bedeutung hat, überführt, und diese in einer weiteren Reaktion mit magnesium-organischen Verbindungen der Formel (IIIb) umsetzt zu den Chlorhydrinen der Formel (XII),

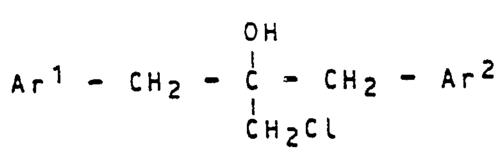 (XII)

in welcher $Ar^1$ und $Ar^2$ die oben angegebene Bedeutung haben.

Die Chlorhydrine der Formel (XII) lassen sich in bekannter Weise mit Basen in die Oxirane der Formel (IV) überführen (vgl. J. Org. Chemistry 27, 2242 [1961]).

Die weiterhin als Ausgangsstoffe zu verwendenden magnesium-organischen Verbindungen (bekannt als « Grignard-Verbindungen ») sind durch die Formeln (IIIa) und (IIIb) allgemein definiert. In diesen Formeln besitzen $Ar^1$ und $Ar^2$ dieselbe vorzugsweise Bedeutung, die bei der Beschreibung der erfindungsgemäßen Stoffe der Formel (I) vorzugsweise für diese Reste genannt wurden. Hal steht vorzugsweise für Chlor oder Brom. Anstelle der magnesium-organischen Verbindungen der Formeln (IIIa) bzw. (IIIb) können auch die entsprechenden alkalimetall-organischen Verbindungen eingesetzt werden.

Die « Grignard-Verbindungen » der Formeln (IIIa) und (IIIb) sind allgemein bekannte Verbindungen

der organischen Chemie.

Die weiterhin als Ausgangsstoffe zu verwendenden Azolylalkalisalze sind durch die Formel (V) allgemein definiert. In dieser Formel besitzt Az dieselbe vorzugsweise Bedeutung, die bei der Beschreibung der erfindungsgemäßen Verbindungen der Formel (I) vorzugsweise für diese Reste genannt wurden. M steht vorzugsweise für Natrium oder Kalium.

Die Alkalisalze von Azolen der Formel (V) sind allgemein bekannt. Sie werden durch Umsetzung von Imidazol bzw. 1,2,4-Triazol mit Natrium- oder Kaliummethylat, oder durch Umsetzung von Imidazol bzw. Triazol mit der äquivalenten Menge des entsprechenden Alkalihydrids erhalten.

Die weiterhin als Ausgangsstoffe zu verwendenden Azolylessigsäureester sind durch die Formel (VI) allgemein definiert. In diesen Formeln besitzt Az dieselbe vorzugsweise Bedeutung, die bei der Beschreibung der erfindungsgemäßen Verbindungen der Formel (I) vorzugsweise für diesen Rest genannt wurden. R steht vorzugsweise für Methyl oder Ethyl.

Die Azolylessigsäureester der Formel (VI) sind bekannt. (vgl. C. Ainsworth, R.G. Jones, J. Am. Chem. Soc. 77, 621-624 (1955)).

Die weiterhin als Ausgangsstoffe zu verwendenden Arylacetonitrile und Arylessigsäureester sind durch die Formeln (VII) bzw. (VIII) allgemein definiert. In diesen Formeln besitzen $Ar^1$ und $Ar^2$ dieselbe vorzugsweise Bedeutung, die bei der Beschreibung der erfindungsgemäßen Verbindungen der Formel (I) vorzugsweise für diese Reste genannt wurden. R steht vorzugsweise für Methyl oder Ethyl.

Die Arylacetonitrile der Formel (VII) und die Arylessigsäure-ester der Formel (IX) sind allgemein bekannte Verbindungen der organischen Chemie oder können in üblichter Art und Weise hergestellt werden.

Als Verdünnungsmittel kommen für die erfindungsgemäßen Verfahren a) und c) inerte organische Lösungsmittel infrage. Hierzu gehören vorzugsweise aliphatische und aromatische Kohlenwasserstoffe, wie z. B. Pentan, Hexan, Petrolether, Ligroin, Benzol, Toluol, Xylol oder auch Ether, wie z. B. Diethylether, Diisopropylether, Tetrahydrofuran oder Dioxan.

Die Reaktionstemperaturen können bei den erfindungsgemäßen Verfahren a) und c) in einem größeren Bereich variiert werden. Im allgemeinen arbeitet man bei Temperaturen zwischen 20 °C und 120 °C, vorzugsweise zwischen 30 °C und 80 °C.

Bei der Durchführung des Verfahrens a) setzt man auf 1 Mol der Verbindung (II) vorzugsweise einen Ueberschuß von 2 bis 3 Mol der Grignard-Verbindung der Formel (IIIb) ein.

Bei der Durchführung des Verfahrens c) setzt man auf ein Mol der Verbindung (VI) vorzugsweise einen Ueberschuß von 2 bis 5 Mol der Grignard-Verbindung der Formel (IIIa) ein.

Die Isolierung der Reaktionsprodukte erfolgt in beiden Fällen in bekannter und üblicher Art und Weise.

Für die erfindungsgemäße Umsetzung nach Verfahren b) kommen als Verdünnungsmittel ebenfalls inerte organische Lösungsmittel infrage. Hierzu gehören vorzugsweise dipolare aprotische Lösungsmittel, wie z. B. Dimethylformamid, Aceton oder Acetonitril.

Die Reaktionstemperaturen können bei Verfahren b) ebenfalls in einem größeren Bereich variiert werden. Im allgemeinen arbeitet man zwischen 20° und 120 °C, vorzugsweise zwischen 30 °C und 100 °C.

Bei der Durchführung des erfindungsgemäßen Verfahrens b) setzt man auf ein Mol substituiertes Oxiran der Formel (IV) im allgemeinen 1,0 bis 2,0 Mol, vorzugsweise 1,0 bis 1,5 Mol Alkalisalz der Formel (V) ein.

Zur Isolierung der Verbindungen der Formel (I) nach Verfahren b) wird das Lösungsmittel abdestilliert, der Rückstand direkt oder nach Aufnahme in einem organischen Solvens mit Wasser gewaschen, die organische Phase über Natriumsulfat getrocknet und im Vakuum von Lösungsmittel befreit.

In einer besondere Ausführungsform des Verfahrens b) stellt man die Ausgangsprodukte der Formel (IV) in einer vorgelagerten Reaktion aus den entsprechenden Chlorhydrinen der Formel (XII) durch Zusatz einer Base her und führt die Umsetzung mit den Alkalisalzen von Azolen der Formel (V) zu den erfindungsgemäßen Verbindungen der Formel (I) ohne Isolierung der als Zwischenprodukte auftretenden Oxirane der Formel (IV) im « Eintopfverfahren » durch.

Zur Herstellung von physiologisch verträglichen Säureadditionssalzen der Verbindungen der Formel (I) kommen vorzugsweise folgende Säuren infrage : Halogenwasserstoffsäuren, wie z. B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z. B. durch Lösen einer Verbindung der Formel (I) in einem geeigneten inerten Lösungsmittel und Hinzufügen der Säure, wie z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise

Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten : Halogenwasserstoffsäuren, wie z. B. Chlorwasserstoffsäure und Bromwasserstoffsäure, ferner Phosphorsäure, Salpetersäure und Schwefelsäure. Die Metallsalzkomplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Mehltau-Arten, wie z. B. gegen den Erreger des echten Getreidemehltaus (Erysiphe graminis) oder gegen den Erreger des echten Apfelmehltaus (Podosphaera leucotricha) eingesetzt werden. Daneben zeigen die erfindungsgemäßen Verbindungen auch eine gute Wirkung gegen die Erreger der Reiskrankheiten Pyricularia oryzae und Pellicularia sasakii.

Bei entsprechenden Aufwandmengen weisen die erfindungsgemäßen Stoffe auch eine wachstumsregulierende Wirkung auf.

Die Wirkstoffe können in die üblichen Formulierungen übergeführt werden, wie Lösungen, Emulsionen, Spritzpulver, Suspensionen, Pulver Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittelals Hilfs lösungsmittel verwendet werden. Als flüssige Lösungsmittel kommen im wesentlichen infrage : Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfratktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen in Frage : z. B. nichtionogene und anionische Emulgatoren, wie Polyoxyethylen-Fettsäureester, Polyoxyethylen-Fettalkohol-ether, z. B. Alkylaryl-polyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

### Beispiel 1

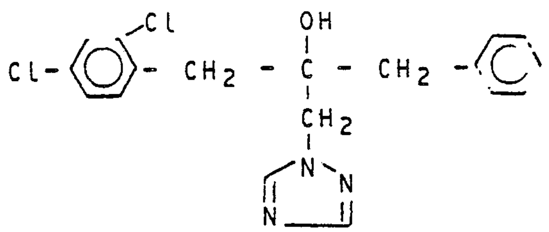

(Verfahren a)

Eine Lösung von 7,5 g (0,027 Mol) 1-(2,4-Dichlorphenyl)-3-(1,2,4-triazol-1-yl)-propan-2-on in 70 ml Tetrahydrofuran wird bei 20 °C bis 25 °C eingetropft in eine Lösung von Benzylmagnesiumchlorid (hergestellt aus 6,8 g (0,054 Mol) Benzylchlorid und 1,3 g (0,054 Mol) Magnesium) in 60 ml Ether. Nach 48 Stunden wird das Lösungsmittelgemisch im Vakuum eingeengt und der Rückstand mit wässriger Ammoniumchloridlösung zersetzt. Man extrahiert die wässrige Lösung mehrfach mit Methylenchlorid, trocknet die vereinigten organischen Extrakte über Natriumsulfat und entfernt das Lösungsmittel im Vakuum. Das verbleibende Oel wird über eine Kieselgelsäule (Laufmittel : Chloroform) chromatographisch gereinigt. Man erhält so 2,9 g (29,7 % der Theorie) an 1-(2,4-Dichlorphenyl)-3-phenyl-2-(1,2,4-triazol-1-yl-methyl)-propan-2-ol vom Schmelzpunkt 126 °C.

Herstellung des Ausgangsproduktes

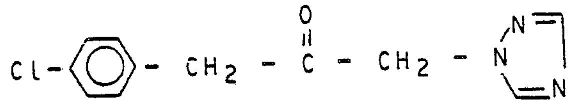

Eine Lösung von 15,5 g (0,1 Mol) 1,2,4-Triazol-1-yl-essigsäure-ethylester in 70 ml Ether wird bei Raumtemperatur eingetropft in eine Lösung von 2,4-Dichlorbenzylmagnesiumchlorid (erhalten aus 58,7 g (0,3 Mol) 2,4-Dichlorbenzylchlorid und 7,3 g (0,3 Mol) Magnesium) in 120 ml Ether. Nach 15 Stunden bei 20 °C bis 25 °C wird das Gemisch mit wässriger Ammoniumchlorid-Lösung zersetzt, die Etherphase abgetrennt, mit Wasser gewaschen, über Natriumsulfat getrocknet und im Vakuum eingedampft. Das verbleibende Oel wird über eine Kieselgelsäule (Laufmittel : Chloroform) chromatographiert.

Man erhält zunächst eine Fraktion, die beim Verrühren mit Ether 3,7 g (8,5 % der Theorie) 1,3-Di-(2,4-dichlorphenyl)-2-(1,2,4-triazol-1-yl-methyl)-propan-2-ol vom Schmelzpunkt 134 °C liefert. Ausden anschließenden Fraktionen erhält man nach Verrühren mit Ether 7,5 g (28 % der Theorie) 1-(2,4-Dichlorphenyl)-3-(1,2,4-triazol-1-yl)-propan-2-on vom Schmelzpunkt 116 °C.

### Beispiel 2

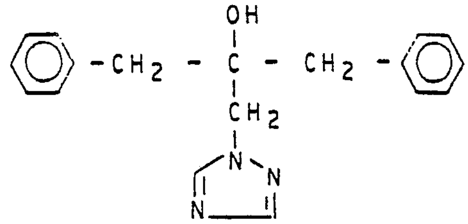

(Verfahren c)

Eine Lösung von 15,5 g (0,1 Mol) 1,2,4-Triazol-1-yl-essigsäureethylester in 50 ml Ether wird bei Raumtemperatur eingetropft in eine Lösung von Benzylmagnesiumchlorid (aus 12,0 g (0,5 Mol) Magnesium und 63,5 g (0,5 Mol) Benzylchlorid) in 150 ml Ether. Nach zwei Tagen (bei 20 °C bis 25 °C) wird das Reaktionsgemisch mit wässriger Ammoniumchlorid-Lösung zersetzt, die Etherlösung abgetrennt, mit Wasser gewaschen, über Natriumsulfat getrocknet und im Vakuum eingedampft. Das verbleibende Oel wird über eine Kieselgelsäule (Laufmittel : Chloroform) chromatographisch gereinigt. Man erhält 16,8 g (57 % der Theorie) 1,3-Diphenyl-2-(1,2,4-triazol-1-ylmethyl)-propan-2-ol vom Brechungsindex $n_D^{20}$ 1,586 5.

In entsprechender Weise und gemäß den angegebenen Verfahren erhält man die folgenden Verbindungen der allgemeinen Formel (I) :

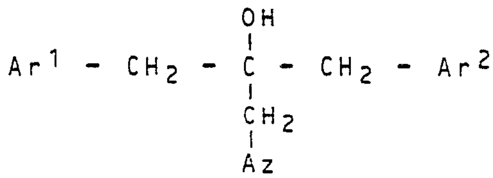

(I)

| Bsp. Nr. | $Ar^1$ | $Ar^2$ | Az | Schmelzpunkt (°C) oder Brechungs-index $n_D^{20}$ |
|---|---|---|---|---|
| 3 | 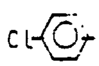 | 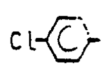 | 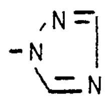 | 125 |
| 4 | 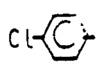 | 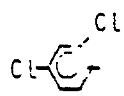 | 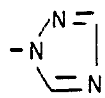 | 150 |
| 5 | 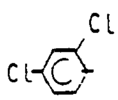 | 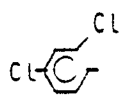 | 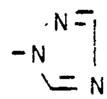 | 134 |
| 6 | 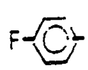 | 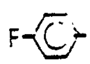 | 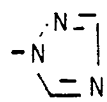 | 1,5348 |
| 7 | 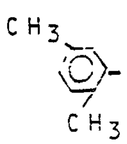 | 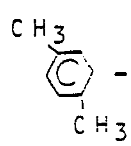 | 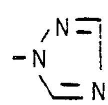 | 134 |
| 8 | 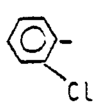 | 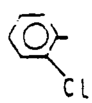 | 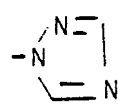 | 138 |

Anwendungsbeispiele

In den nachfolgenden Anwendungsbeispielen werden die folgenden Verbindungen als Vergleichssubstanzen eingesetzt :

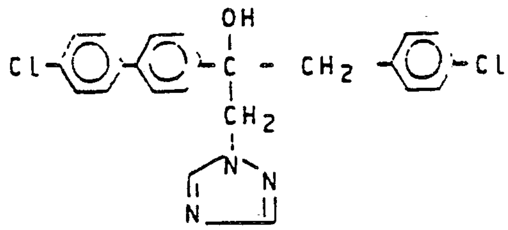

(A)

2-(4-Chlorbiphenyl-4'-yl)-3-(4-chlorphenyl)-1-(1,2,4-triazol-1-yl)-propan-2-ol

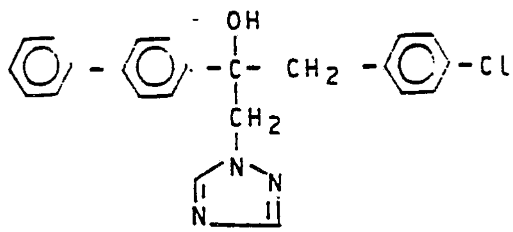

(B)

2-Biphenyl-4-yl-3-(4-chlorphenyl)-1-(1,2,4-triazol-1-yl)-propan-2-ol

Beispiel A

Erysiphe-Test (Gerste) / protektiv

Lösungsmittel : 100 Gewichtsteile Dimepthylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 1, 2, 3 und 6.

Beispiel B

Podosphaera-Test (Apfel) / protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23 °C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 4 und 2.

**Patentansprüche**

1. Azolylbutanole der allgemeinen Formel (I),

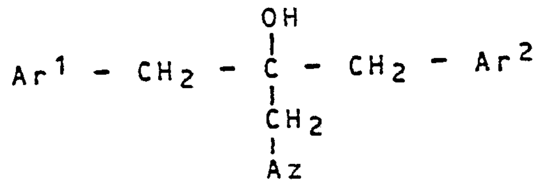

(I)

in welcher

Az für 1,2,4-Triazol-1-yl oder Imidazol-1-yl steht und

$Ar^1$ und $Ar^2$ unabhängig voneinander für Phenyl stehen, wobei die Phenylreste gegebenenfalls ein- bis dreifach substituiert sein können durch Halogen, Alkyl oder Alkoxy mit jeweils 1 bis 4 Kohlenstoffatomen, Halogenalkyl mit 1 bis 2 Kohlenstoffatomen und 1 bis 5 Halogenatomen, sowie durch gegebenenfalls ein- bis dreifach gleich oder verschieden durch Alkyl mit 1 bis 4 Kohlenstoffatomen oder Halogen substituiertes Phenyl,

sowie deren pflanzenverträgliche Säureadditions-Salze und Metallsalz-Komplexe.

2. Verbindungen der allgemeinen Formel (I) in Anspruch 1, wobei

Az für 1,2,4-Triazol-1-yl oder Imidazol-1-yl steht und

$Ar^1$ und $Ar^2$ unabhängig voneinander für Phenyl stehen, welches gegebenenfalls ein- bis dreifach gleich oder verschieden substituiert sein kann durch Fluor, Chlor, Brom, Methyl, Ethyl, Isopropyl, Methoxy, Ethoxy, Isopropyloxy und Trifluormethyl sowie durch gegebenenfalls durch Chlor oder Methyl ein- bis dreifach gleich oder verschieden substituiertes Phenyl.

3. Verfahren zur Herstellung von Azolylbutanolen der allgemeinen Formel (I)

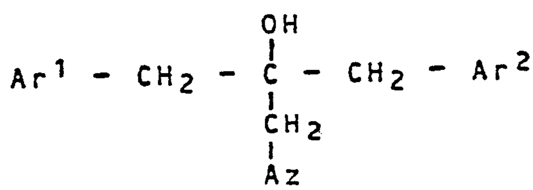

(I)

in welcher

Az für 1,2,4-Triazol-1-yl oder Imidazol-1-yl steht und

$Ar^1$ und $Ar^2$ unabhängig voneinander für Phenyl stehen, wobei die Phenylreste gegebenenfalls ein- bis dreifach substituiert sein können durch Halogen, Alkyl oder Alkoxy mit jeweils 1 bis 4 Kohlenstoffatomen, Halogenalkyl mit 1 bis 2 Kohlenstoffatomen und 1 bis 5 Halogenatomen, sowie durch gegebenenfalls ein- bis dreifach gleich oder verschieden durch Alkyl mit 1 bis 4 Kohlenstoffatomen oder Halogen substituiertes Phenyl,

sowie von deren pflanzenverträglichen Säureadditions-Salzen und Metallsalz-Komplexen, dadurch gekennzeichnet, daß man

a) Azolylpropanone der allgemeinen Formel (II),

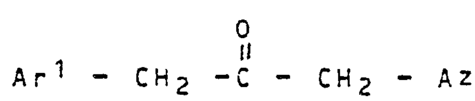

(II)

in welcher $Ar^1$ und Az die oben angegebene Bedeutung haben, mit magnesium-organischen Verbindungen der allgemeinen Formel (III b)

$$Ar^2\text{—}CH_2\text{—}Mg\text{—}Hal \qquad \text{(III b)}$$

in welcher

$Ar^2$ die oben angegebene Bedeutung hat und

Hal für Halogen steht,

in Gegenwart eines Verdünnungsmittels umsetzt, oder

b) substituierte Oxirane der allgemeinen Formel (IV),

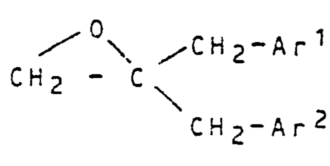

(IV)

in welcher $Ar^1$ und $Ar^2$ die oben angegebene Bedeutung haben, mit Alkalisalzen von Azolen der allgemeinen Formel (V),

$$Az^{\ominus}\ M^{\oplus} \qquad (V)$$

in welcher
Az die oben angegebene Bedeutung hat und
M für ein Alkalimetall steht,
in Gegenwart eines Verdünnungsmittels umsetzt, oder

c) Azolylessigsäureester der allgemeinen Formel (VI),

$$Az—CH_2—COOR \qquad (VI)$$

in welcher
Az die oben angegebene Bedeutung hat und
R für Alkyl steht,
mit magnesium-organischen Verbindungen der Formel (III a),

$$Ar^1—CH_2—Mg—Hal \qquad (III\ a)$$

in welcher
$Ar^1$ die oben angegebenen Bedeutung hat und
Hal für Halogen steht,
in Gegenwart eines Verdünnungsmittels umsetzt, und gegebenenfalls noch an die so erhaltenen Verbindungen der Formel (I) anschließend eine Säure oder ein Metallsalz addiert.

4. Pflanzenschutzmittel, gekennzeichnet durch einen Gehalt an mindestens einem Azolylbutanol der Formel (I) in Anspruch 1 bzw. einem pflanzenverträglichen Säureadditions-Salz oder Metallsalz-Komplex einer Verbindung der Formel (I).

5. Fungizide Mittel, gekennzeichnet durch einen Gehalt an mindestens einem Azolylbutanol der Formel (I) in Anspruch 1 bzw. einem pflanzenverträglichen Säureadditions-Salz oder Metallsalz-Komplex einer Verbindung der Formel (I).

6. Verfahren zur Bekämpfung von Pilzen, dadurch gekennzeichnet, daß man Azolylbutanole der Formel (I) in Anspruch 1 bzw. deren pflanzenverträgliche Säureadditions-Salze oder Metallsalz-Komplexe einer Verbindung der Formel (I) auf Pilze oder ihren Lebensraum einwirken läßt.

7. Verwendung von Azolylbutanolen der Formel (I) in Anspruch 1 bzw. von deren pflanzenverträglichen Säureadditions-Salzen oder Metallsalz-Komplexen einer Verbindung der Formel (I) als Pflanzenschutzmittel.

8. Verwendung von Azolylbutanolen der Formel (I) in Anspruch I bzw. von deren pflanzenverträglichen Säureadditions-Salzen oder Metallsalz-Komplexen der Formel (I) zur Bekämpfung von Pilzen.

9. Verfahren zur Herstellung von Pflanzenschutzmitteln, dadurch gekennzeichnet, daß man Azolylbutanole der Formel (I) in Anspruch 1 bzw. deren pflanzenverträgliche Säureadditions-Salze oder Metallsalz-Komplexe mit Streckmitteln und/oder oberflächenaktiven Mitteln vermischt.

## Claims

1. Azolylbutanols of the general formula (I),

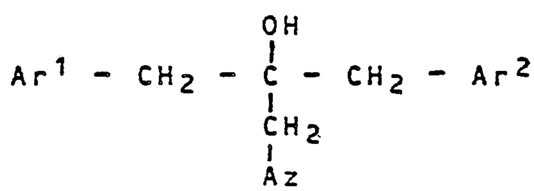

(I)

in which
Az represents 1,2,4-triazol-1-yl or imidazol-1-yl and
$Ar^1$ and $Ar^2$ independently of one another represent phenyl, it being possible for the phenyl radicals optionally to be mono- to trisubstituted by halogen, alkyl or alkoxy with in each case 1 to 4 carbon atoms, halogenoalkyl with 1 to 2 carbon atoms and 1 to 5 halogen atoms, and by phenyl which is optionally mono- to trisubstituted by identical or different substituents from the group comprising alkyl with 1 to 4 carbon atoms and halogen,

and their plant-tolerated acid addition salts and metal salt complexes.

2. Compounds of the general formula (I) in Claim 1, wherein

Az represents 1,2,4-triazol-1-yl or imidazol-1-yl and

$Ar^1$ and $Ar^2$ independently of one another represent phenyl which can optionally be mono- to trisubstituted by identical or different substituents from the group comprising fluorine, chlorine, bromine, methyl, ethyl, isopropyl, methoxy, ethoxy, isopropyloxy and trifluoromethyl and by phenyl which is optionally mono- to trisubstituted by identical or different substituents from the group comprising chlorine and methyl.

3. Process for the preparation of azolylbutanols of the general formula (I)

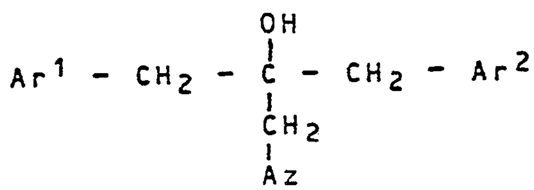

(I)

in which

Az represents 1,2,4-triazol-1-yl or imidazol-1-yl and

$Ar^1$ and $Ar^2$ independently of one another represent phenyl, it being possible for the phenyl radicals optionally to be mono- to trisubstituted by halogen, alkyl or alkoxy with in each case 1 to 4 carbon atoms, halogenoalkyl with 1 to 2 carbon atoms and 1 to 5 halogen atoms, and by phenyl which is optionally mono- to trisubstituted by identical or different substituents from the group comprising alkyl with 1 to 4 carbon atoms and halogen,

and their plant-tolerated acid addition salts and metal salt complexes, characterised in that

a) azolylpropanones of the general formula (II),

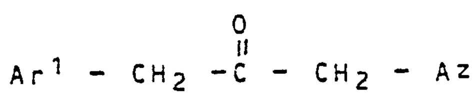

(II)

in which $Ar^1$ and Az have the abovementioned meaning, are reacted with organomagnesium compounds of the general formula (III b)

$$Ar^2—CH_2—Mg—Hal \qquad \text{(III b)}$$

in which

$Ar^2$ has the abovementioned meaning and

Hal represents halogen,

in the presence of a diluent or

b) substituted oxiranes of the general formula (IV),

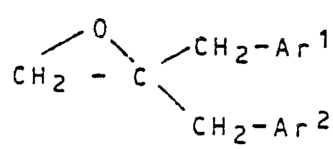

(III)

in which $Ar^1$ and $Ar^2$ have the abovementioned meaning, are reacted with alkali metal salts of azoles of the general formula (V),

$$Az^{\ominus}\ M^{\oplus} \qquad \text{(V)}$$

in which

Az has the abovementioned meaning and

M represents an alkali metal,

in the presence of a diluent, or

c) azolylacetic acid esters of the general formula (VI),

$$Az—CH_2—COOR \qquad \text{(VI)}$$

in which

Az has the abovementioned meaning and
R represents alkyl,
are reacted with organomagnesium compounds of the formula (III a),

$$Ar^1—CH_2—Mg—Hal \quad (III\ a)$$

in which
Ar[1] has the abovementioned meaning and
Hal represents halogen,
in the presence of a diluent, and an acid or a metal salt is then optionally added on to the compounds of the formula (I) thus obtained.

4. Plant protection agents, characterised in that they contain at least one azolylbutanol of the formula (I) in Claim 1 or a plant-tolerated acid addition salt or metal salt complex of a compound of the formula (I).

5. Fungicidal agents, characterised in that they contain at least one azolylbutanol of the formula (I) in Claim 1 or a plant-tolerated acid addition salt or metal salt complex of a compound of the formula (I).

6. Process for combating fungi, characterised in that azolylbutanols of the formula (I) in Claim 1 or their plant-tolerated acid addition salts or metal salt complexes of a compound of the formula (I) are allowed to act on fungi or their environment.

7. Use of azolylbutanols of the formula (I) in Claim 1 or their plant-tolerated acid addition salts or metal salt complexes of a compound of the formula (I) as plant protection agents.

8. Use of azolylbutanols of the formula (I) in Claim 1 or their plant-tolerated acid addition salts or metal salt complexes fo the formula (I) for combating fungi.

9. Process for the preparation of plant protection agents, characterised in that azolylbutanols of the formula (I) in Claim 1 or their plant-tolerated acid addition salts or metal salt complexes are mixed with extenders and/or surface-active agents.

## Revendications

1. Azolylbutanols de formule générale (I)

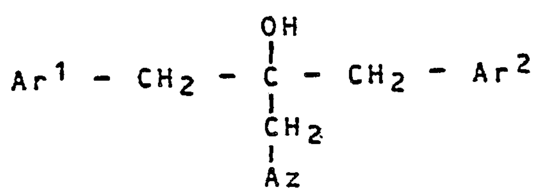

(I)

dans laquelle
Az représente un 1,2,4-triazol-1-yle ou un imidazol-1-1yle et
Ar[1] et Ar[2] représentent indépendamment l'un de l'autre un phényle, les radicaux phényle pouvant éventuellement être substitués une à trois fois par de l'halogène, un alcoyle ou un alcoxy ayant chacun 1 à 4 atomes de carbone, un halogénoalcoyle ayant 1 à 2 atomes de carbone et 1 à 5 atomes d'halogène, de même que par un phényle éventuellement substitué une à trois fois, de manière identique ou différente, par un alcoyle ayant 1 à 4 atomes de carbone ou par de l'halogène,
de même que leurs sels d'addition d'acides et complexes avec des sels métalliques compatibles avec les plantes.

2. Composés de formule générale (I) selon la revendication 1, dans lesquels
Az représente un 1,2,4-triazol-1-yle ou imidazol-1-yle et
Ar[1] et Ar[2] représentent indépendamment l'un de l'autre un phényle qui peut éventuellement être substitué une à trois fois, de manière identique ou différente, par du fluor, chlore, brome, méthyle, éthyle, isopropyle, méthoxy, éthoxy, isopropyloxy et trifluorométhyle, de même que par un phényle éventuellement substitué une à trois fois, de manière identique ou différente, par du chlore ou un méthyle.

3. Procédé de fabrication d'azolylbutanols de formule générale (I) :

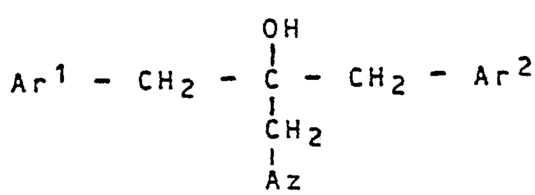

(I)

dans laquelle

Az représente un 1,2,4-triazol-1-yle ou imidazol-1-yle et

$Ar^1$ et $Ar^2$ représentent indépendamment l'un de l'autre un phényle, les radicaux phényle pouvant être éventuellement substitués une à trois fois par de l'halogène, un alcoyle ou un alcoxy ayant chacun 1 à 4 atomes de carbone, par un halogénoalcoyle ayant 1 à 2 atomes de carbone et 1 à 5 atomes d'halogène, de même que par un phényle éventuellement substitué une à trois fois, de manière identique ou différente, par un alcoyle ayant 1 à 4 atomes de carbone ou par de l'halogène,

de même que de leurs sels d'addition d'acides et complexes avec des sels métalliques compatibles avec les plantes, caractérisé en ce que

a) on fait réagir des azolylpropanones de formule générale (II),

$$Ar^1 - CH_2 - \overset{O}{\overset{\|}{C}} - CH_2 - Az \qquad \text{(II)}$$

dans laquelle $Ar^1$ et Az ont la signification indiquée plus haut, avec des composés organo-magnésiens de formule générale (III b) :

$$Ar^2—CH_2—Mg—Hal \qquad \text{(III b)}$$

dans laquelle

$Ar^2$ a la signification indiquée plus haut et

Hal représente de l'halogène,

en présence d'un diluant, ou

b) en ce qu'on fait réagir des oxiranes substitués de formule générale (IV),

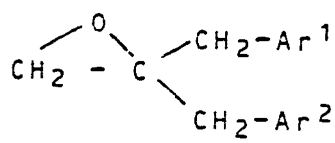

(IV)

dans laquelle $Ar^1$ et $Ar^2$ ont la signification indiquée plus haut, avec des sels alcalins d'azols de formule générale (V),

$$Az^{\ominus} M^{\oplus} \qquad \text{(V)}$$

dans laquelle

Az a la signification indiquée plus haut et

M représente un métal alcalin,

en présence d'un diluant, ou

c) en ce qu'on fait réagir un ester azolylacétique de formule générale (VI),

$$Az—CH_2—COOR \qquad \text{(VI)}$$

dans laquelle

Az a la signification indiquée plus haut et

R représente un alcoyle,

avec des composés organo-magnésiens de formule (III a),

$$Ar^1—CH_2—Mg—Hal \qquad \text{(III a)}$$

dans laquelle

$Ar^1$ a la signification indiquée plus haut et

Hal représente de l'halogène,

en présence d'un diluant, et en ce qu'éventuellement on fixe ensuite un acide ou un sel métallique sur les composés ainsi obtenus de formule (I).

4. Agents de protection des plantes, caractérisés par une teneur en au moins un azolylbutanol de formule (I) selon la revendication 1 ou en un sel d'addition d'acide ou complexe avec un sel métallique compatible avec les plantes d'un composé de formule (I).

5. Agents fongicides, caractérisés par une teneur en au moins un azolylbutanol de formule (I) selon la revendication 1 ou en un sel d'addition d'acide ou complexe avec un sel métallique compatible avec les plantes d'un composé de formule (I).

6. Procédé pour combattre les champignons, caractérisé en ce qu'on fait agir sur les champignons ou sur leur espace vital des azolylbutanols de formule (I) selon la revendication 1 ou des sels d'addition d'acides ou complexes avec des sels métalliques compatibles avec les plantes d'un composé de formule (I).

7. Utilisation d'azolylbutanols de formule (I) selon la revendication 1 ou des sels d'addition d'acides ou complexes avec des sels métalliques compatibles avec les plantes d'un composé de formule (I) comme agents de protection des plantes.

8. Utilisation d'azolylbutanols de formule (I) selon la revendication 1 ou de leurs sels d'addition d'acides ou complexes avec des sels métalliques compatibles avec les plantes de formule (I) pour combattre les champignons.

9. Procédé de fabrication d'agent de protection des plantes, caractérisé en ce qu'on mélange des azolylbutanols de formule (I) selon la revendication 1, ou leurs sels d'addition d'acides ou complexes avec des sels métalliques compatibles avec les plantes, avec des diluants et/ou avec des agents tensioactifs.